# NEUE EUROPÄISCHE PATENTSCHRIFT

(11) **EP 1 197 201 B2**
(45) Veröffentlichungstag und Bekanntmachung des Hinweises auf die Entscheidung über den Einspruch: **16.08.2017**
(45) Hinweis auf die Patenterteilung: 17.01.2007
(21) Anmeldenummer: 01119284.6
(22) Anmeldetag: 10.08.2001
(51) Int. Cl.: A61K 8/92, A61Q 5/06

(54) **Haarwachsprodukt mit Wachsen, nicht-flüchtigen Ölen und flüchtigen, hydrophoben Stoffen**
Wax product for hair with non-volatile oils and volatile hydrophobic substances
Produit de coiffure à base de cire avec des huiles non volatils et des substances hydrophobes volatiles

(30) Priorität: 04.10.2000 DE 10049147
(43) Veröffentlichungstag der Anmeldung: 17.04.2002
(73) Patentinhaber: The Procter & Gamble Company, Cincinnati, OH 45202 (US)
(72) Erfinder: Stein, Bernd, 63768 Hösbach (DE); Franzke, Michael, 64380 Rossdorf (DE); Baecker, Sabine, 65428 Rüsselsheim (DE)
(74) Vertreter: Briatore, Andrea

(56) Entgegenhaltungen:
- EP-A- 0 779 351
- EP-A- 0 795 317
- EP-A- 0 868 898
- EP-A- 0 997 139
- EP-A2- 0 795 317
- EP-B1- 0 868 898
- JP-A- H11 124 316
- JP-A- 2000 239 131
- US-A- 3 330 730
- US-A- 4 001 391
- US-A- 5 286 475
- US-A- 6 066 316

## Beschreibung

Gegenstand der Erfindung ist ein Wachsprodukt zur Behandlung oder Erstellung der menschlichen Frisur. Das Wachsprodukt enthält eine Zusammensetzung mit einem Gehalt an Wachsen oder wachsartigen Stoffen, nicht-flüchtigen, bei Raumtemperatur flüssigen, hydrophoben Ölen sowie leicht-flüchtigen, hydrophoben, bei Raumtemperatur flüssigen oder gasförmigen Stoffen.

Stylingwachs-Zusammensetzungen sind bekannte Produkte zur Haarbehandlung. Sie finden insbesondere Anwendung, um kurzes bis mittellanges Haar trendgerecht in Form zu bringen und der Frisur Halt, Stand und Festigung sowie Glanz zu verleihen. Auch lassen sich mit Harwachsen Konturen und Texture in der Frisur erzeugen. Herkömmliche Haarwachse werden üblicherweise in Tiegeln angeboten und beruhen auf folgendem Wirkungsprinzip: Die Entnahme der Produktmasse erfolgt mit den Fingern. Das Wachs wird in den Handflächen verteilt und durch die Handwärme aufgeschmolzen oder zumindest stark erweicht. Durch die Erweichung oder Aufschrnelzung wird die Einarbeitung des ansonsten zu festen Wachses in das Haar ermöglicht. Im erweichten oder mehr oder weniger flüssigen Zustand wird das Wachs in das Haar eingearbeitet. Im Haar kühlt es ab und erreicht wieder seine Ausgangskonsistenz. Dabei erhärtet es und die gestaltete Frisur erhält Stabilität und Halt sowie häufig einen leichten Wet-Look. Durch dieses Wirkprinzip sind den Produktleistungen der herkömmlichen Stylingwachsprodukte enge Grenzen gesetzt. Damit sich das Wachs gut einarbeiten lässt, darf es bei der Entnahme mit der Hand nicht zu hart sein und der Schmelz- oder Erweichunggspunkt muss in der Nähe der Körpertemperatur liegen. Andererseits läßt sich mit derartigen, weichen Wachsen nur eine mäßige Produktleistung hinsichtlich Stand der Haare, Halt und Volumen der Frisur erreichen. Ausserdem ist die Belastung der Haare vergleichsweise hoch. Mit einer härteren Wachszusammensetzung liesse sich zwar eine bessere Festigung und ein besserer Halt erzielen, aber je härter das Wachs ist, umso härter ist auch die Produktmasse und umso schlechter kann diese verarbeitet und in die Haare eingearbeitet werden. Die Aufgabe bestand also darin, ein Haarwachsprodukt zu entwickeln, welches der Frisur verbesserten Halt und Stand verleiht, wobei aber gleichzeitig die Produktmasse gut verarbeitbar und gut in das Haar einarbeitbar ist.

Ein weiterer Nachteil herkömmlicher Tiegelwachs-Produkte ist, dass bei der Anwendung die Wachsmasse in der Regel mit den Fingern aus einem Tiegel entnommen wird, was zu Verunreinigungen des im Tiegel verbleibenden Restproduktes führen kann. Eine weitere Aufgabe bestand darin, eine Applikationsform für Haarwachse zu finden, bei denen eine Verunreinigung der Produktmasse vermieden wird.

Die EP 795 317 A2 beschreibt bestimmte Wachskombinationen und diese Wachskombinationen enthaltende kosmetische Mittel. Die Wachskombination besteht aus Apfelwachs, Orangen- oder Zitronenwachs und Jojobaöl. Die Mittel können mit einem Treibmittel oder mittels einer mechanisch betriebenen Sprühvorrichtung versprüht werden. Die EP 868 898 A1 beschreibt haarkosmetische Zusammensetzungen in Form von Mikrodispersionen (fine dispersion) von Wachs in Wasser. Die Wachsdispersionen können zusätzlich dispergierte Ölparlikel enthalten. Die Zusammensetzungen können als Aerosolschäume mit Treibgas vorliegen. Die EP 997 139 A beschreibt filmbildende kosmetische Zusammensetzungen zur Erzeugung wasserfester Filme. Die Zusammensetzungen enthalten ein flüchtiges Kohlenwasserstofföl und ein bestimmtes Polyolefinwachs. Zusätzlich können weitere Wachse sowie nichtflüchtige Öle enthalten sein.

Es wurde nun gefunden, dass die Anforderungen erfüllt werden durch ein Wachsprodukt zur Behandlung oder Erstellung der menschlichen Frisur, welches eine Zusammensetzung enthält mit einem Gehalt an Wachsen oder wachsartigen Stoffen, nicht-flüchtigen, bei Raumtemperatur flüssigen, hydrophoben Ölen sowie leichtflüchtigen, hydrophoben, bei Raumtemperatur flüssigen odergasförmigen Stoffen. Das erfindungsgemäße Haarwachsprodukt liegt in Form eines Aerosol-Sprühproduktes vor.

Durch den Einsatz von leicht-flüchtigen Substanzen wie z.B. Pentan, Isododekan o.ä. kann ein weiches, gut zu verarbeitendes und gut in das Haar einarbeitbares Wachs erhalten werden, welches durch Verdunsten der leichtflüchtigen Bestandteile auf den behandelten Haaren aushärtet. Hierdurch ergibt sich ein im Vergleich zu herkömmlichen Haarwachsen verbesserter Stand, mehr Volumen und weniger Belastung. Durch Variation von Art und Anteil der leichtflüchtigen Substanzen kann eine in der Ausgangskonsistenz zähe, wachsartige Zusammensetzung (Tiegelwachs) bis flüssige, versprühbare Zusammensetzung (Sprühwachs) hergestellt werden. Durch Einsatz von gasförmigen Substanzen (Treibgasen wie Propan, Butan o.ä.) können Wachse mit besonders großer Oberfläche (Wachsschnee) hergestellt werden, die sich trotz starker Festigung ebenfalls leicht verarbeiten und leicht in das Haar einarbeiten lassen.

Gegenstand der Erfindung ist ein Aerosol Sprüh-Wachsprodukt gemäß den Patentansprüchen 1 bis 6. Unter Raumtemperatur wird in der Regel eine Temperatur von 20°C verstanden.

Die Begriffe "Wachs", "wachsartig", "wachsförmig" etc. beziehen sich insbesondere auf die Definition für Wachse gemäß Ullmanns Encyklopädie der technischen Chemie, 4. Auflage, Band 24, Seite 3. Demnach sind Wachse bei 20°C knetbar, fest bis brüchig hart, grobbis feinkristallin, durchscheinend bis opak, jedoch nicht glasartig, über 40°C ohne Zersetzung schmelzend, schon wenig oberhalb ihres Schmelzpunktes verhältnismäßig niedrigviskos, stark temperaturabhängig in Konsistenz und Löslichkeit und unter leichtem Druck polierbar.

Klassische Haarwachse sind in organischen Lösungsmitteln löslich oder zumindest suspendierbar. Zu diesen Lösungsmitteln zählen auch die im Aerosolbereich eingesetzten verflüssigbaren Treibgase wie z.B. Propan, Butan und deren Isomere sowie die bei Raumtemperatur unter Normaldruck flüssigen, leicht flüchtigen Alkane wie z.B. Pentan und dessen Isomere. Wird eine Haarwachszusammensetzung zusammen mit einem dieser Treibgase gelöst oder suspendiert und in einer Aerosoldose abgefüllt, so läßt sie sich je nach Sprühkopf entweder fein versprühen oder wie ein Sprühschaum als Wachsschnee ausbringen. Ein kompakterer Wachsschnee (frozen wax) entsteht, wenn man statt des Sprühkopfes einen Schaumkopf verwendet. Der kompakte Wachsschnee entsteht durch Wärmeentzug beim Verdampfen des Treibgases. Die dabei entstehende schaum- bzw. flockenartige Konsistenz mit großer Oberfläche des Wachses ermöglicht eine sehr gute Verteilbarkeit in den Händen und auf den Haaren.

Das sich bei dem erfindungsgemäßen Haarwachsprodukt um ein Aerosol Sprüh-Wachsprodukt handelt, weist die wachshaltige Zusammensetzung bei Raumtemperatur (20°C) eine flüssige, versprüh- oder verschäumbare Konsistenz auf. Die Wachse bzw. wachsartigen Stoffe (A) sind in dem Aerosol Sprüh-Wachsprodukt in einer Menge von 5 bis 30 Gew.%, besonders bevorzugt von 10 bis 20 Gew.% enthalten. Das flüssige, hydrophobe Öl (B) liegt vorzugsweise in einer Menge von 5 bis 30 Gew.%, besonders bevorzugt von 10 bis 20 Gew.% vor. Der leicht-flüchtige hydrophobe Stoff (C) liegt in einer Menge von 45 bis 75 Gew.% vor. Gegenüber herkömmlichen Haarwachsprodukten ist ein Teil der nicht-flüchtigen Öle durch leicht-flüchtige, gasförmige hydrophobe Stoffe ersetzt.

Für Aerosol Sprüh-Wachsprodukte ist der leicht-flüchtige hydrophobe Stoff (C) ein verflüssigbares Treibgas, d.h. er ist bei Raumtemperatur unter Normaldruck gasförmig und unter Druck bei Raumtemperatur ver flüssigbar, ausgewählt aus Propan, n-Butan, Isobutan, fluorierten Kohlenwasserstoffen wie z.B. 1,1-Difluorethan oder 1,1,1,2-Tetrafluorethan. Diese Treibgase können einzeln oder im Gemisch eingesetzt werden, z.B. ein Gemisch von Propan und Butan. Besonders bevorzugt ist ein Gemaisch von Propan und Butan. Als leicht-flüchtiger hydrophober Stoff (C) ist zusätzlich zu den genannten Treibgasen noch ein bei Raumtemperatur flüssiger Stoff mit einem Siedepunkt im Bereich von 30 bis 100°C, vorzugsweise von 35 bis 70 °C enthalten. Geeignet sind beispielsweise flüssige Kohlenwasserstoffe, flüssige cyclische oder lineare Silikone (Dimethylpolysiloxane) oder Gemische der genannten Stoffe. Geeignete Kohlenwasserstoffe sind insbesonders lineare oder verzweigte Alkane mit 5 bis 7 C-Atomen, besonders bevorzugt ist Pentan. Ein geeignetes flüssiges, leicht-flüchtiges Silikon ist z.B. Hexamethyldisiloxan.

Die druckfeste Aerosolverpackung des erfindungsgemäßen Aerosol Sprüh-Wachsproduktes kann aus einem beliebigen, für Aerosol Sprüh- oder Schaumprodukte bekanntem Material gefertigt sein. Geeignete Materialien sind insbesondere Metalle wie Aluminium oder Weissblech. Als Sprüh-oder Schaumköpfe können für das erfindungsgemäße Aerosol Sprüh-Wachsprodukt handelsübliche Sprühköpfe und handelsübliche Schaumköpfe verwendet werden.

### Wachs oder wachsartiger Stoff (A)

Als Wachs oder wachsartiger Stoff (A) kann prinzipiell jedes im Stand der Technik bekannte Wachs eingesetzt werden. Hierzu zählen tierische, pflanzliche, mineralische und synthetische Wachse, mikrokristalline Wachse, makrokristalline Wachse, feste Paraffine, Petrolatum, Vaseline, Ozokerit, Montanwachs, Fischer-Tropsch-Wachse, Polyolefinwachsez.B. Polybuten, Bienenwachs, Wollwachs und dessen Derivate wie z.B. Wollwachsalkohole, Candelillawachs, Carnaubawachs, Japanwachs, gehärtete Fette, Fettsäureester und Fettsäureglyceride mit einem Erstarrungspunkt von jeweils oberhalb 40°C, Polyethylenwachse und Silikonwachse. Die Wachse oder wachsartigen Stoffe haben einen Erstarrungspunkt oberhalb 40°C, vorzugsweise oberhalb 55 °C. Die Nadelpenetrationszahl (0,1 mm, 100 g, 5 s, 25°C; nach DIN 51 579) liegt vorzugsweise im Bereich von 2 bis 70, insbesondere von 3 bis 40. Vorzugsweise ist mindestens ein Wachs enthalten, welches eine Nadelpenetrationszahl kleiner 40, besonders bevorzugt kleiner 20 aufweist. Besonders bevorzugt ist Carnaubawachs und ein Ceresin mit einer Nadelpenetrationszahl kleiner 20 sowie deren Gemisch.

### Nicht-flüchtiges, hydrophobes Öl (B)

Nicht-flüchtige, hydrophobe Öle (B) haben einen Schmelzpunkt von unterhalb 25°C und einen Siedepunkt von über 250 °C, vorzugsweise über 300 °C. Hierfür kann prinzipiell jedes dem Fachmann allgemein bekannte Öl eingesetzt werden. In Frage kommen pflanzliche oder tierische Öle, Mineralöle, Silikonöle oder deren Mischungen. Geeignete Silikonöle sind Polydimethylsiloxane, phenylierte Silikone, Polyphenylmethylsiloxane, Phanyltrimethicone, Poly(C1-20) alkylsiloxane, Alkylmethylsiloxane. Geeignet sind weiterhin Kohlenwasserstofföle, z.B. Paraffin- oder Isoparaffinle, Squalan, Öle aus Fettsäuren und Polyolen, insbesondere Triglyceride. Geeignete pflanzliche Öle sind z.B. Sonnenblumenöl, Kokosöl, Rizinusöl, Lanolinöl, Jojobaöl, Maisöl, Sojaöl. Besonders bevorzugt sind Kohlenwasserstofföle, insbesonders Mineralöle (Paraffinum liquidum).

### Zusätzliche Emulgatoren

In einer bevorzugten Ausführungsform enthält das erfindungsgemäße Wachsprodukt zusätzlich mindestens einen Emulgator, um die Wiederauswaschbarkeit der Zusammensetzung aus dem Haar zu verbessern. Die Emulgatoren sind vorzugsweise in einer Menge von 0,5 bis 20 Gew.%, besonders bevorzugt von 3 bis 15 Gew.% enthalten. Bevorzugte Emulgatoren sind solche aus der Klasse der nichtionischen Tenside. Geeignet sind z.B.
- Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
- C12- bis C22-Fettsäuremono- und -diester von Anlagerungsprodukten von 1 bis 30 mol Ethylenoxid an Glycerin und
- Anlagerungsprodukte von 5 bis 60 mol Ethylenoxid an Rizinusöl oder an gehärtetes (hydriertes) Rizinusöl.
- Mono-, Di- und/oder Triester der Phosphorsäure mit Anlagerungsprodukten von 2 bis 30 mol Ethylenoxid an C8-bis C22-Fettalkohole.

In einer besonders bevorzugten Ausführungsform haben die Emulgatoren wachsartige Konsistenz und einen Tropfpunkt oberhalb 25°C.

Zusätzlich zu den vorstehend genannten Inhaltsstoffen können die erfindungsgemäßen Produkte weitere, übliche kosmetische Zusatzstoffe enthalten:
- Lösungsmittel wie Wasser oder ein-oder mehrwertige C1-bis C4-Alkohole, insbesondere Ethanol, Propanol, Glycerine oder Glykole in einer Menge bis zu 10 Gew.%, vorzugsweise 0,1 bis 8 Gew.%
- Kosmetische Farbstoffe in einer Menge bis zu 6 Gew.%, vorzugsweise 0,1 bis 4 Gew.%, z.B. C.I. Pigment Red 4 (C.I. 12 085), C.I. Pigment Green (C.I. 74 260), und/oder C.I. Vat Blue 4 (C.I. 69 800)
- Perlglanzpigmente in einer Menge von bis zu 25 Gew.%, vorzugsweise 1 bis 20 Gew.%, z.B. solche auf Titandioxid/ Mica-Basis
- Parfüm- und Duftstoffe in einer Menge von bis zu 2 Gew.%, vorzugsweise 0,01 bis 1 Gew.%
- Konservierungsmittel in einer Menge von bis zu 1 Gew.%, vorzugsweise 0,01 bis 0,5 Gew.%, insbesondere Parahydroxybenzoesäureester, Benzoesäure, Salicylsäure, Sorbinsäure, Mandelsäure, Polyhexamethylenbiguanid-hydrochlorid oder Isothiazolinonderivate
- Filmbildende Polymere wie z.B. Polyvinylpyrrolidon oder Vinylpyrrolidon/Vinylacetat Copolymere in einer Menge von bis zu 5 Gew.%, vorzugsweise 0,1 bis 4 Gew.%
- Haarpflegende Zusätze wie z.B. Betain in einer Menge von bis zu 5 Gew.%, vorzugsweise 0,01 bis 4 Gew.%.

### Herstellung

Erfindungsgemäße Sprühprodukte können hergestellt werden, indem die Komponenten ohne die leichtflüchtigen Stoffe (C) aufgeschmolzen und vermischt werden. Anschließend wird auf Raumtemperatur abgekühlt. Die Masse wird dann in dem leichtflüchtigen, flüssigen Stoff gelöst oder suspendiert. Diese Lösung bzw. Suspension wird in die Sprühverpackung abgefüllt und abschließend das Treibgas zugegeben. Die Aerosolverpackung wird entweder mit einem Sprühkopf zur Erzeugung eines Wachssprays oder mit einem Schaumkopf zur Erzeugung eines Wachsschnees versehen.

Die nachfolgenden Beispiele sollen den Gegenstand der Erfindung näher erläutern.

### Beispiele

| **Beispiel** | Aerosol-Wachs |
|---|---|
| 13,0 g | Paraffinum Liquidum |
| 10,5 g | Polycerin^{®} 1894 cs (Ceresin, Penetrationshärte 10-16 bei 25°C) |
| 3,5 g | Carnauba Wachs |
| 3,5 g | Triceteareth-4 Phosphat |
| 3,5 g | PEG-40 Hydrogenated Castor Oil |
| 0,6 g | Parfüm |
| 0,4 g | Propylparaben |
| 35,0 g | Pentan |
| 24,0 g | Butan |
| 5,1 g | Isobutan |
| 0,9 g | Propan |

a) Die Zusammensetzung wird in eine Aerosoldose abgefüllt und mit einem handelsüblichen Sprühkopf versehen. Bei der Abgabe aus der Aerosolverpackung wird ein fein verteiltes, oberflächenreiches Wachsspray erhalten.
b) Die Zusammensetzung wird in eine Aerosoldose abgefüllt und mit einem handelsüblichen Schaumkopf versehen. Bei der Abgabe aus der Aerosolverpackung wird ein kompakter, oberflächenreicher Wachsschnee erhalten.

## Patentansprüche

1. Aerosol Sprüh-Wachsprodukt bestehend aus einer druckfesten Aerosolverpackungseinheit mit einem Sprüh- oder einem Schaumkopf sowie aus einer versprüh- oder verschäumbaren Zusammensetzung enthaltend
(A) 5 bis 30 Gew.-% mindestens eines Wachses und/oder eines wachsartigen Stoffs,
(B) 5 bis 35 Gew.-% mindestens eines nicht-flüchtigen, bei Raumtemperatur flüssigen, hydrophoben Öls und
(C) 45 bis 75 Gew.-% mindestens eines leicht-flüchtigen, hydrophoben Stoffes, wobei Komponente (C) ein Gemisch aus
- einem verflüssigbaren Treibgas ausgewählt aus Propan, n-Butan, Isobutan, Fluorkohlenwasserstoffen oder einem Gemisch davon, und
- einem bei Raumtemperatur flüssigen Stoff mit einem Siedepunkt in einem Bereich von 30 bis 100°C
ist.

2. Wachsprodukt nach einem der vorgenannten Ansprüche, **dadurch gekennzeichnet, dass** das Wachs oder der wachsartige Stoff (A) ausgewählt ist aus Stoffen, welcher bei 25°C eine Nadelpenetrationszahl nach DIN 51579 von kleiner 40 aufweist.

3. Wachsprodukt nach einem der vorgenannten Ansprüche, **dadurch gekennzeichnet, dass** das nicht-flüchtige hydrophobe Öl (B) ausgewählt ist aus Mineralölen, Paraffinölen, Isoparaffinen und Kohlenwasserstoffen oder deren Gemischen, wobei die Öle einen Schmelzpunkt von unterhalb 25°C und einen Siedepunkt von über 250°C aufweise.

4. Wachsprodukt nach einem der vorgenannten Ansprüche, **dadurch gekennzeichnet, dass** die Zusammensetzung zusätzlich einen Emulgator enthält.

5. Wachsprodukt nach Anspruch 4, **dadurch gekennzeichnet, dass** der Emulgator ausgewählt ist aus
a) Anlagerungsprodukten von 2 bis 30 mol Ethylenoxid und/oder 1 bis 5 mol Propylenoxid an C₈- bis C₂₂-Fettalkohole, an C₁₂- bis C₂₂-Fettsäuren oder an Alkylphenole mit 8 bis 15 C-Atomen in der Alkylgruppe,
b) C₁₂₋ bis C₂₂-Fettsäuremono- und -diestem von Anlagerungsprodukten von 1 bis 30 mol Ethylenoxid an Glycerin,
c) Anlagerungsprodukten von 5 bis 60 mol Ethylenoxid an Rizinusöl oder an gehärtetes Rizinusöl,
d) Mono-, Di- und/oder Triestern der Phosphorsäure mit Anlagerungsprodukten von 2 bis 30 mol Ethylenoxid an C₈- bis C₂₂-Fettalkohole
oder aus Gemischen dieser Emulgatoren.

6. Wachsprodukt nach einem der Ansprüche 4 oder 5, **dadurch gekennzeichnet, dass** der Emulgator in einer Menge von 3 bis 20 Gew.-% enthalten ist.

## Claims

1. An aerosol spray wax product consisting of a pressure-resistant aerosol packaging unit with a spray head or a foam head as well as a sprayable or foamable composition containing
(A) 5 to 30 % by weight of at least one wax and/or one waxy substance,
(B) 5 to 35 % by weight of at least one nonvolatile hydrophobic oil, which is liquid at room temperature, and
(C) 45 to 75 % by weight of at least one highly volatile hydrophobic substance, wherein component (C) is
- a liquefiable propellant selected from propane, n-butane, isobutane, fluorocarbons, or a mixture thereof, and
- a substance liquid at room temperature with a boiling point ranging from 30 to 100 °C.

2. The wax product according to any of the preceding claims, **characterized in that** the wax or the waxy substance (A) is selected from substances which have a needle penetration number of less than 40 at 25 °C in accordance with DIN 51579.

3. The wax product according to any of the preceding claims, **characterized in that** the nonvolatile hydrophobic oil (B) is selected from mineral oils, paraffin oils, isoparaffins, and hydrocarbons, or mixtures thereof, wherein the oils have a melting point of less than 25 °C and a boiling point of greater than 250 °C.

4. The wax product according to any of the preceding claims, **characterized in that** the composition additionally contains an emulsifier.

5. The wax product according to claim 4, **characterized in that** the emulsifier is selected from
a) addition products of from 2 to 30 mol ethylene oxide and/or 1 bis 5 mol propylene oxide added to C₈ to C₂₂ fatty alcohols, to C₁₂ to C₂₂ fatty acids, or to alkyl phenols with 8 to 15 C atoms in the alkyl group;
b) C₁₂ to C₂₂ fatty acid monoesters and diesters of addition products of from 1 to 30 mol ethylene oxide added to glycerol;
c) addition products of from 5 to 60 mol ethylene oxide added to castor oil or hydrogenated castor oil;
d) monoesters, diesters, and/or triesters of phosphoric acid with addition products of from 2 to 30 mole ethylene oxide added to C₈ to C₂₂ fatty alcohols; or from mixtures of these emulsifiers.

6. The wax product according to either of claims 4 or 5, **characterized in that** the emulsifier is contained in a quantity of from 3 to 20 % by weight.

## Revendications

1. Produit à base de cire en aérosol à pulvériser, constitué d'une unité d'emballage d'aérosol résistant à la pression, présentant une tête de pulvérisation ou de moussage ainsi que d'une composition pulvérisable ou moussable, contenant
(A) 5 à 30 % en poids au moins d'une cire et/ou d'une substance de type cire,
(B) 5 à 35 % en poids au moins d'une huile hydrophobe non volatile, liquide à température ambiante et
(C) 45 à 75 % en poids au moins d'une substance hydrophobe, volatile, dans lequel le composant (C) est un mélange constitué par
- un gaz propulseur liquéfiable, choisi parmi le propane, le n-butane, l'isobutane, les hydrocarbures fluorés ou un mélange de ceux-ci et
- une substance liquide à température ambiante présentant un point d'ébullition dans une plage de 30 à 100 °C.

2. Produit à base de cire selon l'une quelconque des revendications précédentes, **caractérisé en ce que** la cire ou la substance de type cire (A) est choisie parmi les substances qui présentent, à 25 °C, un indice de pénétration d'une aiguille selon la norme DIN 51579 inférieur à 40.

3. Produit à base de cire selon l'une quelconque des revendications précédentes, **caractérisé en ce que** l'huile (B) hydrophobe non volatile est choisie parmi les huiles minérales, les huiles paraffiniques, les isoparaffines et les hydrocarbures ou leurs mélanges, les huiles présentant un point de fusion inférieur à 25 °C et un point d'ébullition supérieur à 250 °C.

4. Produit à base de cire selon l'une quelconque des revendications précédentes, **caractérisé en ce que** la composition contient en outre un émulsifiant.

5. Produit à base de cire selon la revendication 4, **caractérisé en ce que** l'émulsifiant est choisi parmi
a) les produits d'addition de 2 à 30 moles d'oxyde d'éthylène et/ou de 1 à 5 moles d'oxyde de propylène sur des alcools gras en C₈-C₂₂, sur des acides gras en C₁₂-C₂₂ ou sur des alkylphénols comprenant 8 à 15 atomes de carbone dans le groupe alkyle,
b) les monoesters et les diesters d'acides gras en C₁₂-C₂₂ de produits d'addition de 1 à 30 moles d'oxyde d'éthylène sur du glycérol,
c) les produits d'addition de 5 à 60 moles d'oxyde d'éthylène sur de l'huile de ricin ou sur de l'huile de ricin hydrogénée,
d) les monoesters, le diesters et/ou les triesters de l'acide phosphorique avec des produits d'addition de 2 à 30 moles d'oxyde d'éthylène sur des alcools gras en C₈-C₂₂
ou les mélanges de ces émulsifiants.

6. Produit à base de cire selon l'une quelconque des revendications 4 ou 5, **caractérisé en ce que** l'émulsifiant est contenu en une quantité de 3 à 20 % en poids.
